# EUROPEAN PATENT SPECIFICATION

(11) **EP 2 394 615 B1**
(45) Date of publication and mention of the grant of the patent: **14.10.2015**
(21) Application number: 11180034.8
(22) Date of filing: 18.08.2006
(51) Int. Cl.: A61F 6/00

(54) **Condom applicator**
Kondomapplikator
Applicateur de préservatifs

(30) Priority: 19.08.2005 NL 1029771
(43) Date of publication of application: 14.12.2011
(62) Divisional of application: 06783895.3
(73) Proprietor: Wingman Condoms B.V., 2629 HH Delft (NL)
(72) Inventor: Breur, Paul, 2645AB Delfgauw (NL); Tunovic, Adnan, 2623 CD DELFT (NL); Kroezen, Marcel, 2623 CD DELFT (NL); Foekema, Raimond Thomas, 2611 CN DELFT (NL)
(74) Representative: EP&C

(56) References cited:
- WO-A-97/00652
- WO-A1-93/21873
- WO-A1-02/069861
- FR-A1- 2 693 652

## Description

The present invention relates to an assembly of a condom applicator and a condom according to the preamble of claim 1.

From FR 2 693 652, on which the preamble of claim 1 is based, a condom applicator is known, which in Figures 3 and 4 discloses a condom applicator comprising a filamentary loop. The loop comprises two arcuate opposing guiding portions which in use are located at the rolled up part of the condom. Between the two guiding portions is provided a retention portion which in use lies on the rolled up part of the condom. At the ends of the loop, handling grips are provided.

WO 93/21873 discloses another condom applicator consisting of an annular frame which has two flanges as gripping means. The annular frame has a slit separating two end portions. The flanges are located at the end portions. The end portions are movable transversely of the plane of the annular frame by means of the flanges.

It is an object of the invention to provide an improved condom applicator/condom assembly.

This object is achieved by an assembly according to claim 1.

According to the invention a condom applicator comprises two hingedly connected halves, each halve comprising a retention means for the rolled up part of the condom and a gripping member for gripping each of the halves for moving them apart. The applicator halves can be hinged with respect to each other around a hinge axis which is parallel with the axis of the condom. The gripping members are preferably diametrically opposed, such that they can be readily gripped by fingers of one hand.

Preferred embodiments are laid down in the dependent claims.

The invention will become more apparent from the following description with reference to the drawing, in which:
Fig. 1 shows a perspective view of a preferred embodiment of a condom applicator according to the invention with a rolled up condom arranged in it,
Fig. 2 shows in a schematic cross section the guiding of a condom in the condom applicator of Fig. 1,
Fig. 3 shows a perspective view of a slightly modified version of the embodiment of the condom applicator of Fig. 1,
Fig. 4 shows a packaged condom applicator of Fig. 1 with a condom prefitted in it,
Fig. 5 shows a perspective view of another embodiment of a condom applicator according to the invention,
Fig. 6a and 6b show a perspective view of yet another embodiment of a condom applicator according to the invention, and
Fig. 7a-7c show how a condom applicator according to the invention is handled in use.

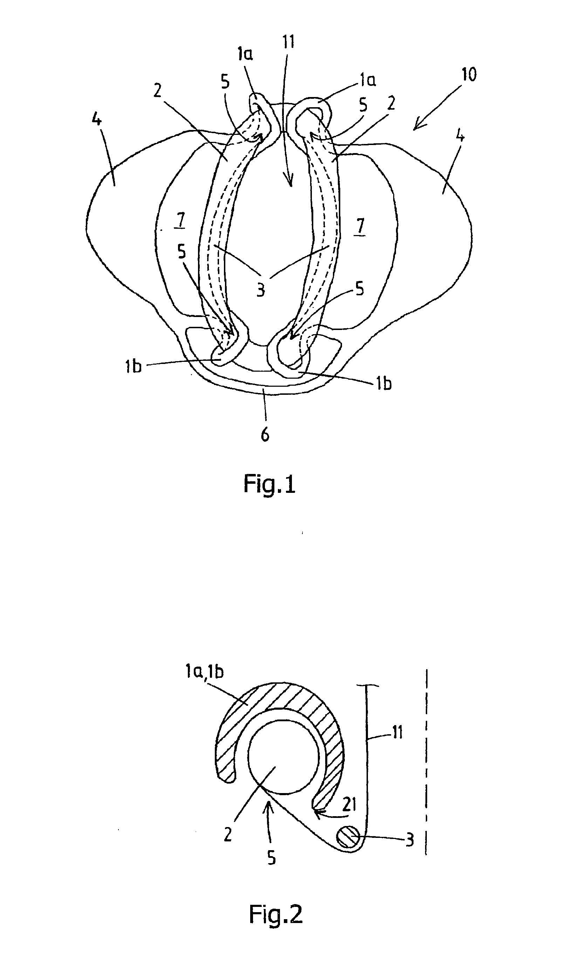

In Fig. 1 a condom applicator is shown which generally is indicated by reference numeral 10. A rolled up condom, indicated by reference numeral 11 is arranged in the condom applicator 10.

The condom applicator 10 has two opposing arcuate guiding members 3. The guiding members 3 in this embodiment are filamentary members with a round, preferably circular cross section. Each of the guiding members may describe an arc of a circle and is arranged such that it is directed with its concave side towards the opposing guiding member 3. Said arc of a circle preferably has a radius of curvature which is substantially greater than the average radius of a condom and the arc extends for example over an angle of 30°**-** 60°, preferably around 45°. This shape provides the advantage that the condom applicator 10 can be used to stretch condoms of different sizes, i.e. with different diameters.

Each end of the guiding members 3 is provided at its respective ends with a receiving loop 1 a and 1b, respectively. The receiving loops 1a and 1b have a dimension such that a rolled up part 2 of the condom 11 in a fully rolled up state can be received in the receiving loops 1 a, 1 b of the respective guiding members 3. The receiving loops 1a, 1b have each an opening 5 which is narrower than the diameter, i.e. the thickness, of the rolled up part 2 of a fully rolled up condom 11.

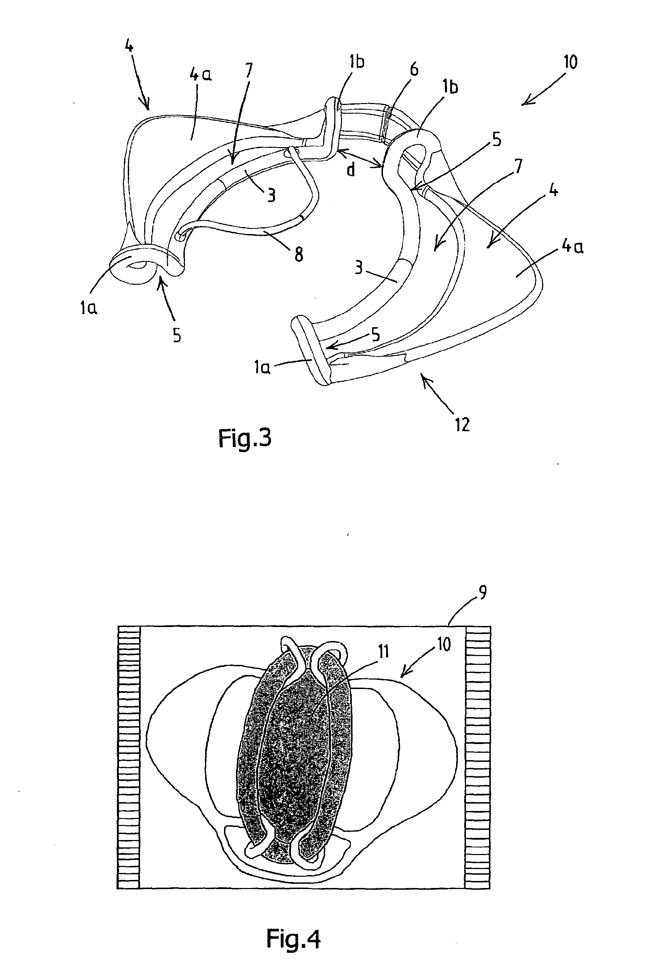

Each of the loops 1 a, 1 b extends from the corresponding guiding member 3 outwardly in a substantially radial direction with respect to the guiding member 3. In other words, each loop 1 a, 1 b lies in a plane which is substantially perpendicular with respect to the longitudinal direction of the corresponding guiding member 3 at the location where the loop 1 a, 1 b is arranged. This is best seen in Fig. 3. In this manner the loops 1a, 1 b can enclose the rolled up part 2 of the condom 11 well and it is assured that the rolled up part of the condom 11 can rotate within the receiving loop 1.

In the preferred embodiment of Fig. 1 and Fig. 3 one end of each receiving loop 1a, 1b is attached to the corresponding guiding member 3. Preferably the receiving loops 1 a, 1 b are formed as an integral part of the guiding members 3. The other end of the receiving loop 1 a, 1 b is attached to a gripping member 4. The gripping member 4 is generally shaped as a ring segment which is situated radially outward from the guiding member. Each gripping member 4 extends substantially concentric with the corresponding guiding member 3. The ring segment can have a gripping tab 4a, which can have any suitable shape. Preferably the gripping tabs 4a are inclined in an upward and outward direction, which provides a better grip for moving the applicator 10 down along the penis. It is also possible to connect the gripping member 4 at another location to the guiding member 3. Between the gripping member 4 and the corresponding guiding member 3 a space 7 is present through which part of the condom 11 extends along the guiding member 3.

The guiding member 3, the receiving loops 1a, 1b attached to its ends and the gripping member 4 attached to the guiding member 3 via the receiving loops 1a, 1 b, constitute one half of the condom applicator 10. The two opposing applicator halves are connected on one side by a hinge element 6, which defines a hinge axis which is parallel to the axis of the condom. The hinge element 6 comprises in the shown embodiment a flexible connection member, which is attached to one end of each of the guiding members 3 at the receiving loops 1a, 1b. The other ends of the guiding members 3 are free, i.e. not connected to each other and can be moved apart. The hinge member 6 is formed from a flexible material. It can be such that it acts as a spring, the spring force of which counteracts the spreading apart of the applicator halves, such that in an unloaded state the free end of the applicator halves are close together as is shown in Fig. 1 and Fig. 3.

The receiving loops 1a, 1b allow the condom 11 to be better retained by the applicator 10, also when the spreaded state of the applicator halves is varied during the application of the condom on a penis. In particular the loops 1a at the free end of the guiding elements 3, i.e. the end opposite to the end where the hinge element 6 is attached, are effective to retain the condom 11 and allow to determine better the moment at which the condom 11 is released by the applicator 10. This contrary to for example the condom applicator disclosed in FR 2 693 652, wherein there are no retention means at the open end of the loop and the condom can inadvertently be released too early from the applicator.

Preferably the loops 1 b, which are at the end of the guiding elements 3 where the hinge element 6 is provided, are in an unloaded state of the applicator 10 spaced apart sufficiently to prevent that the condom 11 can get clamped between them, in particular when the applicator 10 is hinged open. The condom material getting stuck beteen the loops 1 b would lead to a less smooth rolling off of the condom 11 and local stretching of the condom material resulting in stretching marks on the rolled of condom. It was found that with regard to the average size of condoms it was preferable to have a distance d (cf. Fig. 3) between the loops 1 b of 0,5 - 0,8 mm to prevent this effect from occurring.

The condom applicator shown in Fig. 3 has a retention loop 8 which is attached with both its ends to one of the guiding members 3. The retention loop 8 is made of a flexible material and extends in the direction of the other guiding member 3 such that in use the teat part of a condom can extend through the retention loop 8 and is folded over it such that the end of the teat part can be laid under the opposing guiding member 3.

The condom applicator 10 in Figs. 1 and 3 are moulded in one piece from a flexible plastic material. A suitable material has sufficient elasticity such that the applicator 10 is elastically deformable, which is advantageous for its use. Furthermore, a suitable material is such that it has a sufficiently low roughness, such that the applicator 10 after the moulding process has a sufficiently smooth surface, which is important to prevent damage of the condom by the applicator 10. Polypropylene (PP) and High Density Polyethylene (HDPE) are found to fulfill the requirements, but also other suitable materials are conceivable.

The condom 11 can be arranged in the applicator 10 by bringing the rolled up condom 11 with its open end turned away from the applicator 10 from the underside (indicated in Fig. 3 by arrow 12) of the applicator 10 towards the applicator 10. The rolled up part 2 of the condom 11 is inserted through the openings 5 into the receiving loops 1a, 1b. By pressing the rolled up part 2 in the openings 5, which are narrower than the diameter of the rolled up part 2, the loops 1 a, 1 b, due to their flexibility deform and the openings become wider so as to allow the rolled up part 2 to pass. After the rolled up part 2 has passed the openings 5, the loops 1 a, 1 b deform back to their rest state, whereby the rolled up part 2 is retained in the four loops 1a, 1 b of the condom applicator 10. The arcuate guiding members 3 are brought under the rolled up part 2 of the condom 11 as can be best seen in Fig. 1.

Although the design allows a user to readily fit a new condom in the applicator 10, it is at this moment envisaged that the condom applicator is a disposable product, which is thrown away after use.

The condom applicator 10 and the condom 11 are preferably delivered in an preassembled state in one package 9 as is shown in Fig. 4, such that the assembly is immediately ready for use after removing the package 9. In this way no preparatory acts are necessary that could disturb the lovemaking.

When the condom is applied over a penis the diameter of the open end of the condom 11 can be increased by spreading the guiding members 3, by means of fingers of one hand gripping the opposed gripping members 4 and moving them apart. While moving down the condom applicator 10 along the penis the guiding members 3 guide the condom 11 over most of the circumference as is illustrated schematically in Fig. 2. Also, the guiding members 3 prevent that the condom 11 rubs against the edge 21 of the respective openings 5 of the loops 1 a, 1 b, which is illustrated in Fig. 2, which could lead to undesirable stretching of the condom material.

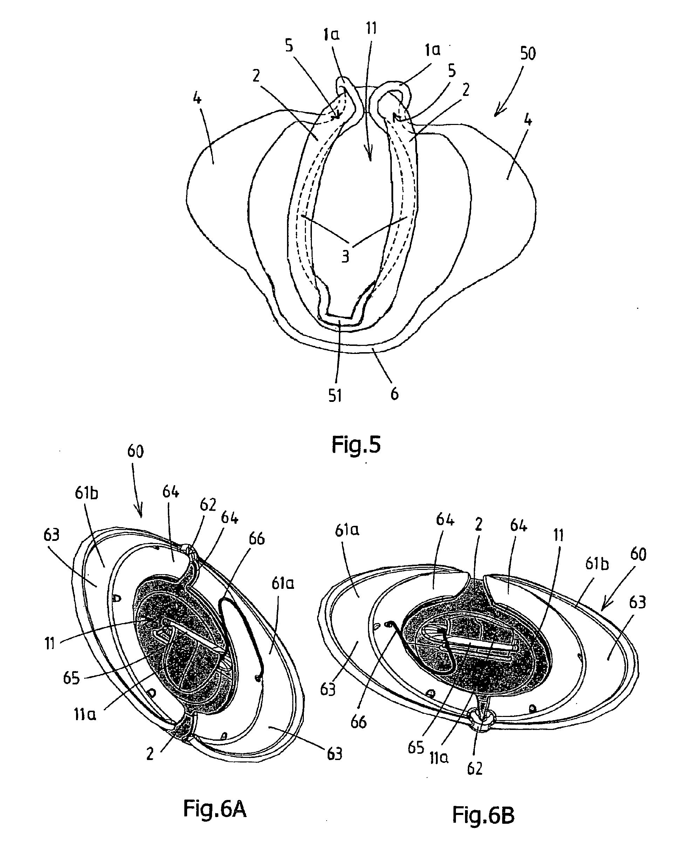

While moving down the applicator 10 along the penis, the rolled up part 2 of the condom 11 rolls within the receiving loops 1 a, 1 b. The working of the loops 1 a, 1 b is comparable with the working of a bearing. Because of the small contact surface of the loops 1 a, 1 b with the rolled up part 2 of the condom 11, a low friction between the condom 11 and the loops 1 a, 1 b is advantageously achieved during rolling off. This contrary to for example condom applicators which have a groove like receiving means (e.g. in Fig. 6) which contacts the rolled up part of the condom over a larger part of the circumference. The possible presence of a lubricant on the condom 11 can advantageously further reduce the bearing friction between the loops 1 a, 1 b and the rolled up part 2 of the condom 11. It is important that the rolled up part 2 of the condom 11 can roll in the loops 1 a, 1 b with a sufficiently low friction so as to prevent that the length rolled off of the rolled up part 2 does not become smaller than the length over which the applicator 10 is moved down along the penis, because this would lead to stretching of the condom material which creates undesired tensions.

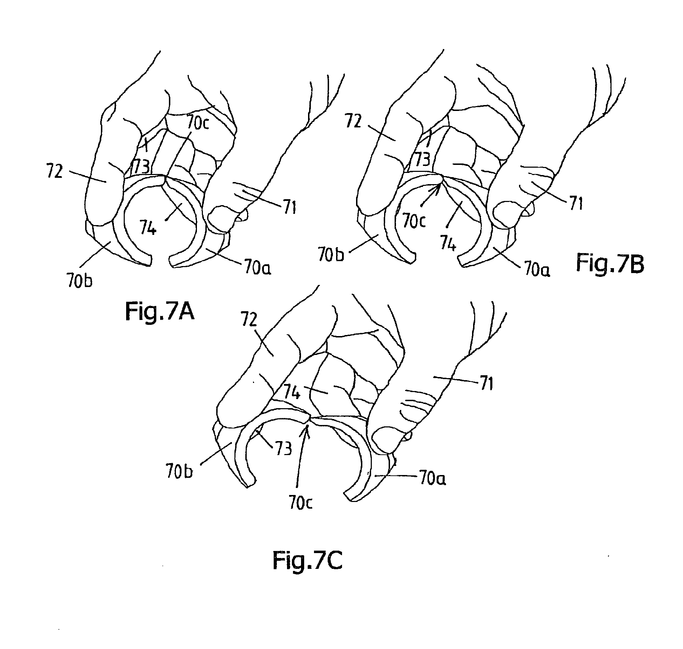

As a consequence of rolling down the rolled up part 2 of the condom 11, the diameter of rolled up part 2 decreases gradually. After the condom 11 has been unrolled over a certain length, the diameter of the rolled up part 2 will be smaller than the width of the respective openings 5 of the respective receiving loops 1a, 1 b, such that the rolled up part 2 can pass through the said openings 5. Thus the applicator 10 can be removed from the condom 11. The handling of the applicator will be described further below with reference to Fig. 7a-7c.

In Fig. 5 a condom applicator 50 is shown which is a variant of the condom applicator of Fig. 1. Therefore corresponding elements are indicated by the same reference numerals. The condom applicator 50 only has loops 1a on one end of the respective guiding members 3. The other ends of the respective guiding members 3 are hingedly connected by a connection member 51. The connection member 51 can be formed as a filamentary member or another flexible member to allow a hinging between the applicator halves. The connection member engages the rolled up part 2 of the condom 11 on its upper side.

Still another embodiment of a condom applicator according to the invention is shown in Figs. 6a and 6b. This condom applicator indicated by reference numeral 60 comprises two applicator halves 61 a and 61 b, which are connected by a hinge member 62. Each applicator half 61 a, 61 b comprises a substantially semicircular ring segment. The radial innermost portion of the half ring has a receiving groove 64 which is open to the underside. In use the receiving groove 64 in the rolled up part 2 of a condom 11 is received. The radially outermost portion 63 of the ring segment constitutes a gripping surface which can be gripped between two fingers. A clip 65 which clamps on the teat portion 11a of the condom 11 is attached to the applicator half 61 b by means of a cord 66 or the like. When applying the condom 11 on a penis the clip 65 will be pulled automatically from the teat portion 11a of the condom 11 by the action of the cord 66 which has only a limited length with respect to the full length of the condom 11.

The handling of the different embodiments of the condom applicator described in the above is now explained in general with reference to Figs. 7a-7c. In Figs. 7a-7c the condom is omitted for the sake of clarity. In Fig. 7a is shown how the gripping member of one applicator half 70a can be clamped between the thumb 71 and the ring finger 74. The gripping member of the other applicator half 70b can be clamped between the index finger 72 and the middle finger 73 of the same hand. Spreading the two mentioned pairs of fingers leads to the spreading of the applicator halves 70a and 70b, which are hingedly connected on one side by a hinge connection 70c.

By spreading the applicator halves 70a, 70b as is shown in Fig. 7b the rolled up part of the condom is stretched, such that it can be applied more easily over the penis and the applicator with the condom can be moved without much resistance in a quick movement downwards.

After the condom is fully rolled over the penis, the applicator halves 70a, 70b can be spread further as is shown in Fig. 7c, whereby on the side opposing the hinge connection an opening can be created which is broad enough for the penis to pass, such that the applicator can be removed in a lateral direction with respect to the penis.

It is noted that, although it is preferred, the gripping means do not necessarily have to be adapted to be gripped by two pairs of fingers. It is also envisaged that for instance an annular member or the like is provided at each applicator half. Each of said annular members is such that a finger can be inserted into it. By spreading the fingers, which are of the same hand, the applicator halves are spread and the same working is achieved as is described hereabove. Of course also other modifications of gripping members are imaginable, as long as the gripping members allow the applicator halves to be handled and be spread by the use of a single hand.

## Claims

1. Assembly of a condom applicator (10, 50, 60) and a condom (11) pre-fitted in the condom applicator (10, 50, 60), said condom applicator (10, 50, 60) comprising two opposing applicator halves (61 a, 61 b, 70a, 70b), each comprising a receiving means (1a, 1b, 64) in which the rolled up part (2) of the condom (11) is received and a gripping member (4, 63) for gripping each of the halves for moving them apart, **characterized in that** the applicator halves are connected on one side by a hinge member (6, 62,70c) defining a hinge axis which is parallel to the axis of the condom (11), and **in that** the gripping members (4, 63) are diametrically opposed with respect to the axis of the condom (11).

2. Assembly according to claim 1, wherein the gripping members (4, 63) are substantially shaped as ring segments which in use extend concentrically with the condom (11).

3. Assembly according to claim 1 or 2, wherein one of the gripping members (4, 63) is adapted to be clamped between the index finger and the middle finger and the other gripping member (4, 63) is adapted to be clamped between the thumb and the ring finger of the same hand.

4. Assembly according to any one of the preceding claims, wherein the receiving means comprises receiving loops (1a, 1 b) in which the rolled up part of the condom is received.

5. Assembly according to any one of the claims 1-3, wherein the receiving means comprises a receiving groove (64) in which the rolled up part of the condom is received.

6. Assembly according to any one of the preceding claims, wherein the hinge member (6) is constituted by a loop of flexible material.

7. Assembly according to any one of the preceding claims, wherein the hinge member (6) has a spring action.

8. Assembly according to any one of the preceding claims, wherein the condom applicator is moulded in one piece from a plastic material.

9. Package (9) containing an assembly according to any one of the preceding claims.

## Patentansprüche

1. Anordnung aus einem Kondomapplikator (10, 50,60) und einem Kondom (11), das in den Kondomapplikator (10, 50,60) voreingepasst ist, wobei der Kondomapplikator (10, 50,60) zwei gegenüberliegende Applikatorhälften (61a, 61b, 70a, 70b) hat, die jeweils ein Aufnahmemittel (1a, 1b, 64), in welches der aufgerollte Teil (2) des Kondoms (11) aufgenommen ist, und ein Greifelement (4,63) aufweisen, um jede der Hälften zu greifen, um sie auseinander zu bewegen, **dadurch gekennzeichnet, dass** die Applikatorhälften an einer Seite durch ein Scharnierelement (6, 62, 70c) verbunden sind, welches eine Scharnierachse definiert, die parallel zur Achse des Kondoms ist (11), und dass die Greifelemente (4,63) diametral entgegengesetzt bezüglich der Achse des Kondoms (11) sind.

2. Anordnung nach Anspruch 1, bei der die Greifelemente (4,63) im Wesentlichen als Ringsegmente geformt sind, die sich bei Verwendung konzentrisch mit dem Kondom (11) erstrecken.

3. Anordnung nach Anspruch 1 oder 2, bei der eines der Greifelemente (4,63) dazu ausgestaltet ist, zwischen dem Zeigefinger und dem Mittelfinger eingeklemmt zu werden, und das andere Greifelement (4,63) dazu ausgestaltet ist, zwischen dem Daumen und dem Ringfinger der gleichen Hand eingeklemmt zu werden.

4. Anordnung nach einem der vorhergehenden Ansprüche, bei der das Aufnahmemittel Aufnahmeschleifen (1a, 1b) aufweist, in welche der aufgerollte Teil des Kondoms aufgenommen ist.

5. Anordnung nach einem der Ansprüche 1 bis 3, bei der das Aufnahmemittel einer Aufnahmenut (64) aufweist, in die der aufgerollte Teil des Kondoms aufgenommen ist.

6. Anordnung nach einem der vorhergehenden Ansprüche, bei der das Scharnierelement (6) durch eine Schleife aus flexiblem Material gebildet wird.

7. Anordnung nach einem der vorhergehenden Ansprüche, bei der das Scharnierelement (6) eine Federwirkung hat.

8. Anordnung nach einem der vorhergehenden Ansprüche, bei welcher der Kondomapplikator in einem Stück aus einem Kunststoffmaterial gegossen ist.

9. Packung (9) mit einer Anordnung nach einem der vorhergehenden Ansprüche.

## Revendications

1. Ensemble composé d'un applicateur de préservatif (10, 50, 60) et d'un préservatif (11) préinstallé sur l'applicateur de préservatif (10, 50, 60), ledit applicateur de préservatif (10, 50, 60) comprenant deux moitiés d'applicateur opposées (61 a, 61 b, 70a, 70b), chacune comprenant un moyen de réception (1a, 1b, 64) dans lequel la partie enroulée (2) du préservateur (11) est reçue et un élément de préhension (4, 63) pour saisir chacune des moitiés afin de les écarter, **caractérisé en ce que** les moitiés d'applicateur sont raccordées sur un côté par un élément de charnière (6, 62, 70c) définissant un axe de charnière qui est parallèle à l'axe du préservatif (11) et **en ce que** les éléments de préhension (4, 63) sont diamétralement opposés par rapport à l'axe du préservatif (11).

2. Ensemble selon la revendication 1, dans lequel les éléments de préhension (4, 63) sont sensiblement formés comme des segments annulaires qui, à l'usage, s'étendent de manière concentrique avec le préservatif (11).

3. Ensemble selon la revendication 1 ou 2, dans lequel l'un des éléments de préhension (4, 63) est adapté pour être serré entre l'index et le majeur et l'autre élément de préhension (4, 63) est adapté pour être saisi entre le pouce et l'annulaire de la même main.

4. Ensemble selon l'une quelconque des revendications précédentes, dans lequel le moyen de réception comprend des boucles de réception (1 a, 1 b) dans lesquelles la partie enroulée du préservateur est reçue.

5. Ensemble selon l'une quelconque des revendications 1 à 3, dans lequel le moyen de réception comprend une rainure de réception (64) dans laquelle la partie enroulée du préservatif est reçue.

6. Ensemble selon l'une quelconque des revendications précédentes, dans lequel l'élément de charnière (6) est constitué par une boucle de matériau souple.

7. Ensemble selon l'une quelconque des revendications précédentes, dans lequel l'élément de charnière (6) a une action de ressort.

8. Ensemble selon l'une quelconque des revendications précédentes, dans lequel l'applicateur de préservatif est moulé d'un seul tenant à partir d'une matière plastique.

9. Emballage (9) contenant un ensemble selon l'une quelconque des revendications précédentes.
